# EUROPEAN PATENT APPLICATION

(11) **EP 4 834 298 A1**
(43) Date of publication of application: **04.11.2026**
(21) Application number: 24913387.7
(22) Date of filing: 26.12.2024
(51) Int. Cl.: A61F 9/007, A61B 90/00, A61B 17/00

(54) **SCLERAL MARKER AND MARKING APPARATUS COMPRISING SAME**

(30) Priority: 27.12.2023 KR 20230193187
(71) Applicant: Seoul National University Hospital, Seoul 03080 (KR)
(72) Inventor: KIM, Min Seok, Seongnam-si, Gyeonggi-do 13620 (KR)
(74) Representative: V.O.
(86) International application number: PCT/KR2024/021096
(87) International publication number: WO 2025/143780

(57) **Abstract**

The present invention provides a scleral marker and a marking apparatus comprising same. The marking apparatus according to an embodiment comprises: a body; a connection part extending from the body in the longitudinal direction of the body; and a marking part coupled to the connection part, wherein the marking part includes a plurality of markers in contact with an eyeball, the markers can be disposed to be spaced apart from each other by a predetermined distance while forming an arc having a curvature corresponding to the eyeball, the plurality of markers comprise a first marker and a second marker, and the first marker and the second marker may be disposed in directions orthogonal to each other when viewed from above.

## Description

### [Technical Field]

The present invention relates to a scleral marker and a marking apparatus including the same, and more particularly, to a scleral marker for marking a point at which a needle is inserted into a sclera, and a marking apparatus including the same.

### CROSS-REFERENCE TO RELATED APPLICATION

This application claims priority to Korean Patent Application No. 10-2023-0193187 filed on December 27, 2023, the entire contents of which are incorporated herein by reference.

Meanwhile, the present invention was made with the support of the following in-house research project under the auspices of Seoul National University Bundang Hospital.
[In-house research project that supported this invention]
[MSRI Project No.] 14-2023-0026
[Research management institution] Seoul National University Bundang Hospital
[Program name] Independent Research in Priority Support Area
[Project name] Adjuvant Effect and Safety Verification and Indication Development of Intraocular Gas Injection (0.3 mL) for Lowering Failure Rate of Conventional Retinal Detachment Surgery
[Contribution ratio] 100%
[Host organization] Seoul National University Bundang Hospital
[Research period] May 1, 2023 to April 30, 2024

### [Background Art]

Rhegmatogenous retinal detachment, which is one of ocular diseases, is a disease in which the retina is detached from the eyeball, and it is a condition requiring emergency surgery. When rhegmatogenous retinal detachment occurs, scleral buckling is performed, in which a fixing member (e.g., a sponge) is fixed by being tied near the position of the retinal break from the outside of the eyeball, thereby reattaching the retina. In scleral buckling, the fixing member is positioned near the position of the retinal break, and needles are inserted at positions spaced apart by a predetermined interval so as to suture and fix the fixing member to the eyeball.

Rhegmatogenous retinal detachment is a disease in which the retina located relatively at the rear of the eyeball is detached. Therefore, when the operator performs scleral buckling, securing a sufficient surgical field for the rear of the eyeball is difficult, and when a conventional surgical instrument such as a caliper is used, access to the rear of the eyeball is not easy, so that inserting a needle at the position where the fixing member is to be sutured is difficult. This results in a longer surgery time, and when a needle is not inserted at an accurate position, it may lead to failure of the surgery.

### [Disclosure]

### [Technical Problem]

An object of the present invention is to provide a scleral marker allowing for efficient surgery, and a marking apparatus including the same.

Another object of the present invention is to provide a scleral marker allowing for easy access to the rear of the eyeball, and a marking apparatus including the same.

Still another object of the present invention is to provide a marker allowing for collectively marking positions at which needles are inserted, and a marking apparatus including the same.

The problems to be solved by the present invention are not limited to the above-mentioned problems, and other problems that are not mentioned herein will be clearly understood by those skilled in the art to which the present invention pertains from the present specification and the accompanying drawings.

### [Technical Solution]

The present invention provides a scleral marker and a marking apparatus including the same. A marking apparatus according to an embodiment may include: a body; a connection part extending from the body in a longitudinal direction of the body; and a marking part coupled to the connection part, wherein the marking part may include a plurality of markers in contact with an eyeball, and the markers may be disposed to be spaced apart from each other by a predetermined distance while forming an arc having a curvature corresponding to the eyeball.

According to an embodiment, the plurality of markers may include a first marker and a second marker, and the first marker and the second marker may be disposed in directions orthogonal to each other when viewed from above.

According to an embodiment, the first marker may be disposed to be spaced apart in the longitudinal direction of the body, and the second marker may be disposed to be spaced apart in a direction perpendicular to the longitudinal direction of the body when viewed from above.

According to an embodiment, the connection part may include: a first connection part extending from one end of the body; and a second connection part extending from the other end of the body, and the marking part may include: a first marker body connected to the first connection part and coupled to the first marker; and a second marker body connected to the second connection part and coupled to the second marker.

According to an embodiment, the first marker body may have a longitudinal direction parallel to the longitudinal direction of the body when viewed from above, and the second marker body may have a longitudinal direction orthogonal to the longitudinal direction of the body when viewed from above.

According to an embodiment, the first marker may be formed at a lower end of the first marker body, and the second marker may be formed at an upper end of the second marker body.

According to an embodiment, the first marker may include a 1-1 marker, a 1-2 marker, and a 1-3 marker, the 1-1 marker may be formed at a front end of the first marker body, the 1-3 marker may be formed at a rear end of the first marker body, the 1-2 marker may be formed between the 1-1 marker and the 1-3 marker, a straight-line distance between a tip of the 1-1 marker and a tip of the 1-2 marker may be 5 mm, and a straight-line distance between the tip of the 1-2 marker and a tip of the 1-3 marker may be 2.5 mm.

According to an embodiment, the second marker may include a 2-1 marker and a 2-2 marker, the 2-1 marker and the 2-2 marker may be respectively formed at both ends of the second marker body, and a straight-line distance between a tip of the 2-1 marker and a tip of the 2-2 marker may be 8 mm.

According to an embodiment, an end of the marker may be formed to be flat.

According to an embodiment, each of the first connection part and the second connection part may be formed in a shape whose diameter decreases with increasing distance from the body.

According to an embodiment, lattice-shaped protrusions may be formed on an outer surface of the body.

According to an embodiment, the apparatus may be used in surgery including scleral buckling performed to reattach a detached retina.

According to an embodiment, the marker may be in contact with the sclera constituting the eyeball to mark positions at which needles are inserted in order to fix a fixing member at a break position.

In addition, the present invention provides a kit. A kit according to an embodiment may include a marking apparatus and a packaging member accommodating the marking apparatus.

### [Advantageous Effects]

According to an embodiment of the present invention, a fixing member can be efficiently sutured at a break position of the retina.

In addition, according to an embodiment of the present invention, the marker can easily access the rear of the eyeball.

In addition, according to an embodiment of the present invention, positions at which needles are inserted can be collectively marked.

In addition, according to an embodiment of the present invention, the positions at which needles are inserted can be accurately marked in accordance with the direction in which the fixing member is to be sutured.

The effects of the present invention are not limited to the above-mentioned effects, and other effects that are not mentioned herein will be clearly understood by those skilled in the art to which the present invention pertains from the present specification and the accompanying drawings.

### [Description of Drawings]

FIG. 1 is a side cross-sectional view schematically showing an eyeball in which rhegmatogenous retinal detachment has occurred and to which a fixing member has been sutured.
FIGS. 2A and 2B are side views schematically showing the eyeball according to the suturing direction of the fixing member.
FIG. 3 is a perspective view schematically showing a marking apparatus according to an embodiment.
FIG. 4 is a side view schematically showing the marking apparatus of FIG. 3.
FIG. 5 is a plan view schematically showing the marking apparatus of FIG. 3.
FIG. 6 is a side view schematically showing an enlarged view of portion A of FIG. 3.
FIG. 7 is a front view schematically showing an enlarged view of portion B of FIG. 3.
FIG. 8 is a perspective view schematically showing a marking apparatus according to another embodiment.

### [Modes of the Invention]

Hereinafter, embodiments of the present invention will be described in detail with reference to the accompanying drawings so that those skilled in the art to which the present invention pertains can easily carry out the present invention. The embodiments of the present invention may be modified and implemented in various forms and are not limited to the embodiments described below. In addition, the embodiments described below are provided so that the present invention can be more completely described to those skilled in the art to which the present invention pertains. Therefore, the shapes and the like of the components in the drawings are exaggerated to emphasize a clearer description. In addition, in describing preferred embodiments of the present invention in detail, when it is determined that a detailed description of related well-known functions or configurations may unnecessarily obscure the gist of the present invention, the detailed description thereof will be omitted. Further, the same reference numerals are used throughout the drawings for parts having similar functions and operations.

The term "comprising" a certain component means that, unless specifically stated to the contrary, other components may be further included rather than being excluded. Specifically, it should be understood that terms such as "comprise" or "have" are intended to designate the presence of features, numbers, steps, operations, components, parts, or combinations thereof described in the specification, and do not preclude in advance the presence or addition of one or more other features, numbers, steps, operations, components, parts, or combinations thereof.

A singular expression includes a plural expression unless the context clearly indicates otherwise. In addition, although terms such as "first" and "second" may be used to describe various components, the components are not limited by these terms. These terms may be used for the purpose of distinguishing one component from another component. For example, without departing from the scope of the present invention, a first component may be named a second component, and similarly, a second component may also be named a first component.

Unless otherwise defined, all terms used herein, including technical and scientific terms, have the same meaning as commonly understood by those skilled in the art to which the present invention pertains. Terms such as those defined in commonly used dictionaries should be interpreted as having meanings consistent with their meanings in the context of the relevant art, and unless clearly defined below, are not to be interpreted in an idealized or overly formal sense.

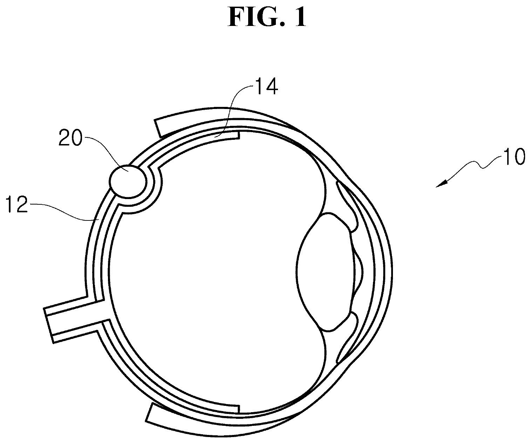

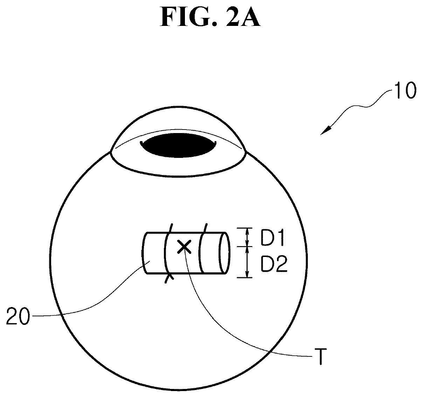

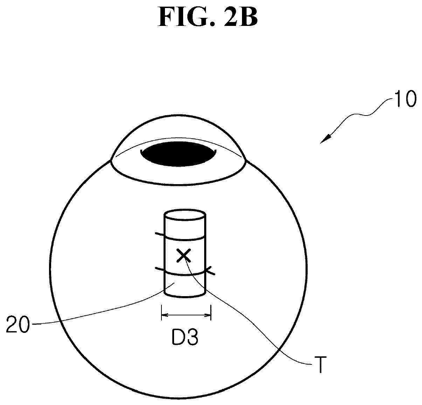

FIG. 1 is a side cross-sectional view schematically showing an eyeball in which rhegmatogenous retinal detachment has occurred and to which a fixing member has been sutured. FIGS. 2A and 2B are side views schematically showing the eyeball according to the suturing direction of the fixing member. The relative sizes and positions of the tissues of the eyeball of FIG. 1 may be somewhat different from those of an actual eyeball and may be represented in a somewhat exaggerated manner. In addition, the size and position of the fixing member 20 shown in FIGS. 2A and 2B may be different from an actual surgical position and may be represented in a somewhat exaggerated manner.

Referring to FIGS. 1, 2A, and 2B, an eyeball 10 may be composed of a sclera 12 and a retina 14. The sclera 12 is located relatively on the outer side among the structures constituting the eyeball so as to entirely surround the eyeball. The retina 14 is located at the rear of the eyeball relative to the pupil, more rearward than the sclera 12. Scleral buckling may be performed to treat the eyeball 10 in which rhegmatogenous retinal detachment, one of retinal diseases, has occurred. In scleral buckling, a fixing member 20 is positioned near the position of the retinal break, and a needle is inserted near the position where the fixing member 20 is positioned to suture and fix the fixing member 20 onto the eyeball. By suturing the fixing member 20 onto the eyeball, the pressure, displacement, and the like of the eyeball structure are increased, thereby restoring and reattaching the detached retina 14 to its original position, in order to treat rhegmatogenous retinal detachment disease.

The fixing member 20 according to an embodiment may be a medical sponge. The sponge may be a silicone sponge product of Mira, Inc. (United States) (e.g., Silicone Sponge-506) and/or FCI S.A.S. (France), which is used when performing scleral buckling. The sizes of the silicone sponge products of Mira, Inc. (United States) and/or FCI S.A.S. (France) may be standardized.

For example, as shown in FIG. 2A, the fixing member 20 may be positioned near a position corresponding to a break position T on the retina of the eyeball 10. In this case, the fixing member 20 may be positioned on and sutured to the eyeball 10 in a state in which a longitudinal direction of the fixing member is oriented in a horizontal direction. Here, the horizontal direction may be understood as a direction substantially orthogonal to a direction toward the pupil when the eyeball 10 is viewed from the side. According to an embodiment, in the scleral buckling in which the fixing member 20 is positioned in the horizontal direction and sutured onto the eyeball 10, the fixing member 20 is positioned so as to cover the break position T, and then the fixing member 20 is to be sutured onto the eyeball at a predetermined interval in a vertical direction. More preferably, needles are inserted at a position spaced upward from the break position T by a first interval D1 and at a position spaced downward from the break position T by a second interval D2, thereby suturing the fixing member 20 onto the eyeball 10. According to an embodiment, the first interval D1 may be approximately 2.5 mm, and the second interval D2 may be approximately 5 mm.

In addition, for example, as shown in FIG. 2B, the fixing member 20 may be sutured onto the eyeball 10 in a state in which a longitudinal direction of the fixing member is oriented in a vertical direction at the break position T on the retina of the eyeball 10. Here, the vertical direction may be understood as a direction substantially parallel to a direction toward the pupil when the eyeball 10 is viewed from the side. According to an embodiment, when the fixing member 20 is sutured onto the eyeball 10 in a state in which the longitudinal direction of the fixing member 20 is oriented in the vertical direction, after the fixing member 20 is positioned so as to cover the break position T, the fixing member 20 is to be sutured onto the eyeball 10 at a predetermined interval in the horizontal direction (e.g., a third interval D3). For example, the third interval D3 may be approximately 8 mm.

The direction of the fixing member 20 sutured onto the eyeball 10 may vary depending on the retinal detachment condition of the patient. In other words, depending on the retinal detachment condition of the patient, the medical staff may position the longitudinal direction of the fixing member 20 in the vertical direction and suture the fixing member, or may position the longitudinal direction of the fixing member 20 in the horizontal direction and suture the fixing member. Hereinafter, for convenience of description, a case in which the longitudinal direction of the fixing member 20 is oriented in the horizontal direction and the fixing member is sutured onto the eyeball 10 is defined as a "horizontal-direction surgery," and a case in which the longitudinal direction of the fixing member 20 is oriented in the vertical direction and the fixing member is sutured onto the eyeball 10 is defined as a "vertical-direction surgery."

However, the above-described examples of the first interval D1, the second interval D2, and the third interval D3 may, of course, vary depending on the size specification of the fixing member 20 (e.g., a sponge).

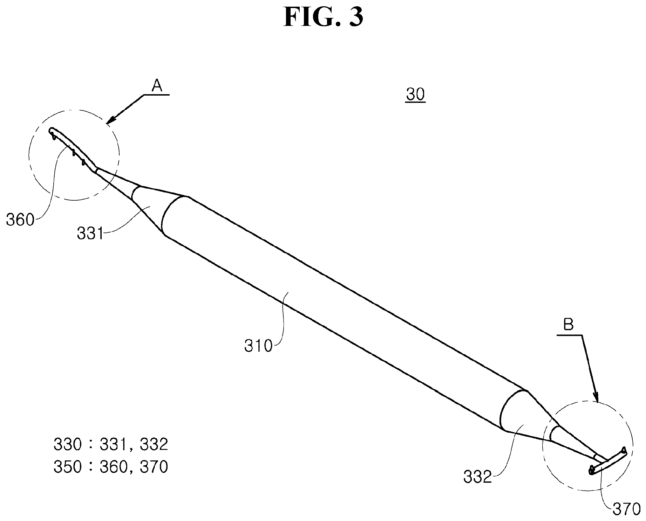

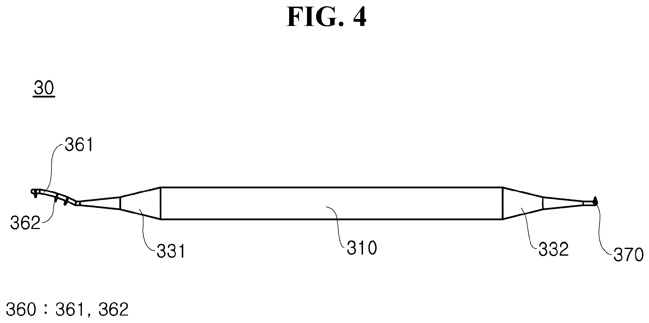

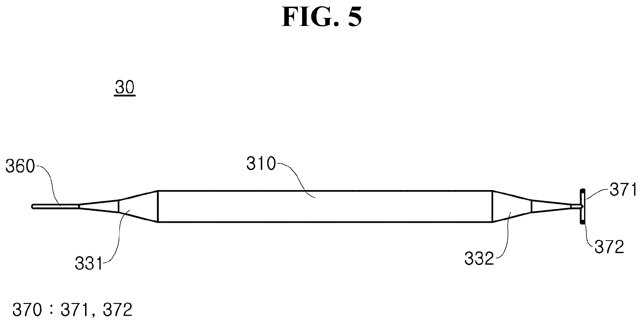

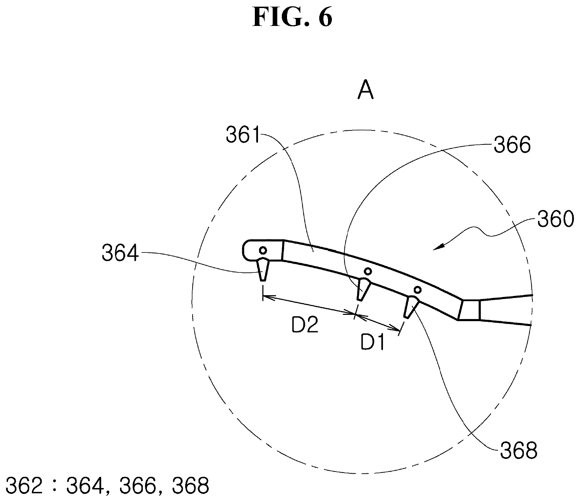

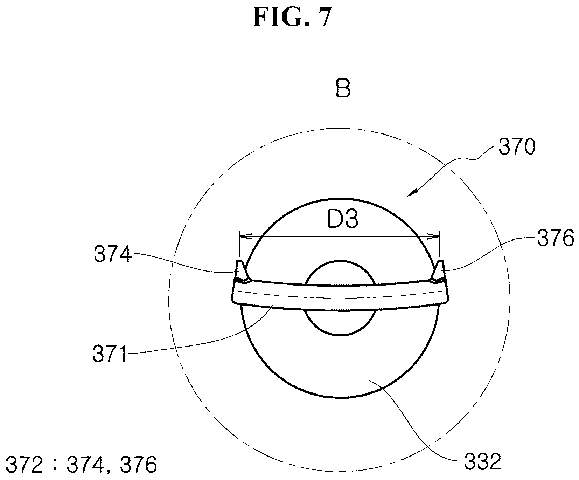

FIG. 3 is a perspective view schematically showing a marking apparatus according to an embodiment. FIG. 4 is a side view of the marking apparatus of FIG. 3, FIG. 5 is a plan view of the marking apparatus of FIG. 3, FIG. 6 is an enlarged view of portion A of FIG. 3, and FIG. 7 is an enlarged view of portion B of FIG. 3. Hereinafter, a marking apparatus according to an embodiment of the present invention will be described in detail with reference to FIGS. 3 to 7 together with FIGS. 1, 2A, and 2B.

A marking apparatus 30 according to an embodiment may be used in scleral buckling. More specifically, the marking apparatus 30 may be used to mark, on the sclera 12, positions at which needles are inserted in order to suture and reattach the fixing member 20 onto the eyeball 10. However, the present invention is not limited thereto, and the marking apparatus 30 according to an embodiment may, of course, be used in various surgeries accompanied by marking on the sclera 12 in order to treat various ocular diseases. Hereinafter, for convenience of understanding, a case in which the marking apparatus 30 according to an embodiment is used in scleral buckling will be described as an example.

The marking apparatus 30 may include a body 310, a connection part 330, and a marking part 350. The body 310, the connection part 330, and the marking part 350 may be integrally formed. However, the present invention is not limited thereto, and the body 310, the connection part 330, and the marking part 350 may be separately formed and coupled to one another in a manner such as bonding. In addition, the body 310, the connection part 330, and the marking part 350 may be made of a material including stainless steel. Without being limited to the above-described example, the body 310, the connection part 330, and the marking part 350 may be manufactured of a combination of highly durable and relatively rigid materials such as titanium, plastic, and polycarbonate, or a combination of other suitable materials.

The body 310 according to an embodiment may be a portion that the operator grips. The body 310 may have a generally rod shape. A cross-section of the body 310 may be circular. However, the present invention is not limited thereto, and the body 310 may have various cross-sections such as a quadrangular or hexagonal cross section. Although not shown, the body 310 may have a shape whose diameter increases from a middle portion toward an end so as to improve the grip feel for the operator. The operator may stably manipulate the marking apparatus 30 by gripping the body 310 with a hand.

The connection part 330 according to an embodiment extends from the body 310 in the longitudinal direction of the body 310. More specifically, the connection part 330 may include a first connection part 331 and a second connection part 332. The first connection part 331 and the second connection part 332 may be formed symmetrically with respect to the body 310. The first connection part 331 extends from one end of the body 310, and the second connection part 332 extends from the other end of the body 310 opposite to the one end of the body 310 in the longitudinal direction of the body 310. The first connection part 331 and the second connection part 332 may be formed in a generally conical shape. In addition, the first connection part 331 and the second connection part 332 may have a shape whose diameter decreases with increasing distance from the body 310. Due to such shapes of the first connection part 331 and the second connection part 332, the operator can easily secure a visual field when performing scleral buckling.

The marking part 350 according to an embodiment may mark positions on the sclera 12 at which needles are inserted when performing scleral buckling. The marking part 350 may include a first marking part 360 and a second marking part 370. According to an embodiment, the first marking part 360 may mark positions on the sclera 12 at which needles are inserted when performing the above-described "horizontal-direction surgery," and the second marking part 370 may mark positions on the sclera 12 at which needles are inserted when performing the above-described "vertical-direction surgery." A more detailed description thereof will be provided below.

The marking part 350 may be connected to the connection part 330. More specifically, the first marking part 360 may be connected to the first connection part 331, and the second marking part 370 may be connected to the second connection part 332.

The first marking part 360 according to an embodiment may include a first marker body 361 and a first marker 362. The first marker body 361 according to an embodiment is connected to the first connection part 331. The first marker body 361 extends from the first connection part 331 in a direction away from the body 310, along a direction generally parallel to the longitudinal direction of the body 310. According to an embodiment, the first marker body 361 has a longitudinal direction parallel to the longitudinal direction of the body 310 when viewed from above.

In addition, the first marker body 361 may be formed to be curved when viewed from the side. In other words, the first marker body 361 may be formed in a generally arc shape when viewed from the side. For example, when viewed from the side, a front end of the first marker body 361 may be positioned higher than a rear end thereof. The first marker body 361 may be formed to have a curvature corresponding to the curvature of the eyeball 10. Here, the front end refers to an end of the first marker body 361 opposite to the rear end, and the rear end refers to an end of the first marker body 361 connected to the first connection part 331.

The first marker 362 according to an embodiment is coupled to the first marker body 361. According to an embodiment, the first marker 362 may be coupled to a lower end of the first marker body 361. A plurality of first markers 362 may be provided. For example, there may be three first markers 362. According to an embodiment, the first marker 362 may include a 1-1 marker 364, a 1-2 marker 366, and a 1-3 marker 368.

The 1-1 marker 364, the 1-2 marker 366, and the 1-3 marker 368 according to an embodiment may each have a truncated cone shape. In other words, the 1-1 marker 364, the 1-2 marker 366, and the 1-3 marker 368 may each have a tip of a flat shape. Each diameter of the tips of the 1-1 marker 364, the 1-2 marker 366, and the 1-3 marker 368 may be approximately 0.2 mm. Medical ink may be applied to each tip surface of the 1-1 marker 364, the 1-2 marker 366, and the 1-3 marker 368 to mark positions on the sclera 12 at which needles are inserted. More specifically, the 1-1 marker 364, the 1-2 marker 366, and the 1-3 marker 368 may mark positions on the sclera 12 at which needles are inserted during the "horizontal-direction surgery." In addition, the lengths between the upper ends and the lower ends of the 1-1 marker 364, the 1-2 marker 366, and the 1-3 marker 368 may all be the same.

The 1-1 marker 364, the 1-2 marker 366, and the 1-3 marker 368 may be disposed to be spaced apart from one another by a predetermined interval and coupled to the lower end of the first marker body 361. Accordingly, the 1-1 marker 364, the 1-2 marker 366, and the 1-3 marker 368 may be disposed to be spaced apart along a direction generally parallel to the longitudinal direction of the body 310 when viewed from above.

The 1-1 marker 364 is formed at the front end of the first marker body 361. The 1-3 marker 368 is formed at the rear end of the first marker body 361. In addition, the 1-2 marker 366 is formed between the 1-1 marker 364 and the 1-3 marker 368. Due to the above-described shape of the first marker body 361, when the 1-1 marker 364, the 1-2 marker 366, and the 1-3 marker 368 are viewed from the side, the 1-1 marker 364 may be positioned higher than the 1-2 marker 366, and the 1-2 marker 366 may be positioned higher than the 1-3 marker 368. In other words, the first markers 362, 364, and 366 may be disposed to be spaced apart while forming an arc so as to have a curvature corresponding to the curvature of the eyeball.

According to an embodiment, a straight-line distance between the tip of the 1-1 marker 364 and the tip of the 1-2 marker 366 may be the second interval D2, and a straight-line distance between the tip of the 1-2 marker 366 and the tip of the 1-3 marker 368 may be the first interval D1. The first interval D1 may be approximately 5 mm, and the second interval D2 may be approximately 2.5 mm. Accordingly, in the scleral buckling in which the fixing member 20 is placed in the horizontal direction and sutured onto the eyeball 10, the first marking part 360 can accurately mark points on the sclera 12 at which needles are to be inserted.

The second marking part 370 according to an embodiment may include a second marker body 371 and a second marker 372. The second marker body 371 according to an embodiment has a longitudinal direction perpendicular to the longitudinal direction of the body 310 when viewed from above. In addition, the second marker body 371 is connected to the second connection part 332. For example, a middle portion of the second marker body 371 is connected to an end of the second connection part 332. In addition, the second marker body 371 may be formed to be curved when viewed from the front. In other words, the second marker body 371 may be formed in a generally arc shape when viewed from the front. For example, when viewed from the front, the second marker body 371 may be formed to be curved such that the middle portion of the second marker body is positioned at a lower height than both ends thereof. The curvature of the second marker body 371 may correspond to the curvature of the eyeball 10.

The second marker 372 according to an embodiment is coupled to the second marker body 371. According to an embodiment, the second marker 372 may be coupled to an upper end of the second marker body 371. A plurality of second markers 372 may be provided, and for example, two markers may be provided. According to an embodiment, the second marker 372 may include a 2-1 marker 374 and a 2-2 marker 376.

The 2-1 marker 374 and the 2-2 marker 376 according to an embodiment may each have a truncated cone shape. The tips of the 2-1 marker 374 and the 2-2 marker 376 may have a flat shape. Each diameter of the tips of the 2-1 marker 374 and the 2-2 marker 376 may be approximately 0.2 mm. Medical ink may be applied to the tip surfaces of the 2-1 marker 374 and the 2-2 marker 376, respectively, to mark positions on the sclera 12 at which needles are inserted. More specifically, the 2-1 marker 374 and the 2-2 marker 376 may mark positions on the sclera 12 at which needles are inserted during the "vertical-direction surgery." In addition, the lengths of the 2-1 marker 374 and the 2-2 marker 376 may all be the same.

The 2-1 marker 374 and the 2-2 marker 376 may be disposed to be spaced apart by a predetermined interval. In addition, the 2-1 marker 374 and the 2-2 marker 376 may each be coupled to the upper end of the second marker body 371. Accordingly, the 2-1 marker 374 and the 2-2 marker 376 may be disposed to be spaced apart along a direction orthogonal to the longitudinal direction of the body 310 when viewed from above. In addition, the arrangement direction of the 2-1 marker 374 and the 2-2 marker 376 may be orthogonal to the arrangement direction of the 1-1 marker 364, the 1-2 marker 366, and the 1-3 marker 368 when viewed from above.

The 2-1 marker 374 and the 2-2 marker 376 may be formed at both ends of the second marker body 371, respectively. The 2-1 marker 374 and the 2-2 marker 376 may be disposed at substantially the same height when viewed from the front. A straight-line distance between the tip of the 2-1 marker 374 and the tip of the 2-2 marker 376 may be the third interval D3. The third interval D3 may be approximately 8 mm. Accordingly, when the fixing member 20 is sutured onto the eyeball 10 in a state of being placed in the vertical direction, the second marking part 370 can accurately mark points on the sclera 12 at which needles are to be inserted.

When performing scleral buckling with the fixing member 20 placed in the horizontal direction, the first marking part 360 can accurately and collectively mark positions on the sclera 12 at which needles are to be inserted. In addition, when performing scleral buckling with the fixing member 20 placed in the vertical direction, the second marking part 370 can accurately and collectively mark positions on the sclera 12 at which needles are to be inserted. In other words, when scleral buckling is performed using the marking apparatus 30 according to an embodiment of the present invention, a retinal break can be reattached with the fixing member 20 placed in either the horizontal direction or the vertical direction using a single apparatus. In other words, by using different markers 362 and 372 according to the fixing direction of the fixing member 20 depending on the retinal detachment condition of the patient, efficient scleral buckling can be performed. In addition, since the intervals of the markers 362 and 372 for marking the needle insertion positions are determined and designed to correspond to the size-standardized fixing member 20, marking can be collectively performed at accurate positions on the sclera 12, thereby shortening the surgery time. Furthermore, since the first marker body 361 and the second marker body 371 are formed to have a curvature corresponding to the curvature of the eyeball, the marking apparatus 30 can smoothly enter up to the break position of the retina at the rear of the eyeball 10 while minimizing secondary damage to the eyeball 10.

Hereinafter, a marking apparatus according to another embodiment of the present invention will be described. Since the marking apparatus described below has substantially the same or similar structure as the marking apparatus described with reference to FIGS. 3 to 7, a description of overlapping content will be omitted.

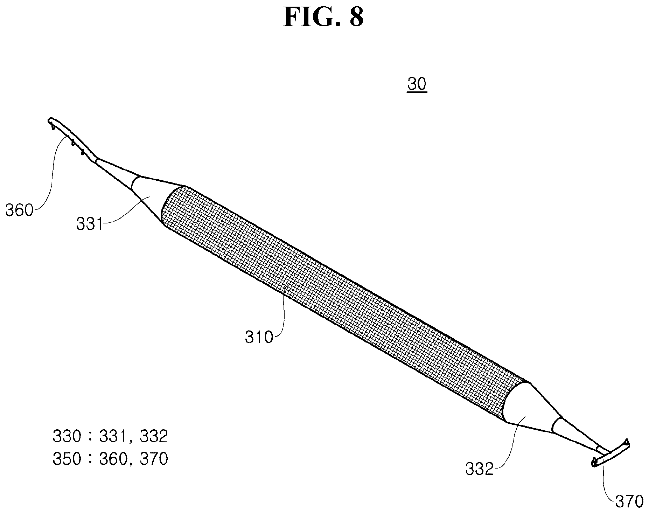

FIG. 8 is a perspective view schematically showing a marking apparatus according to another embodiment.

Referring to FIG. 8, protrusions may be formed on an outer surface of the body 310 according to an embodiment. The protrusions formed on the outer surface of the body 310 may be arranged in a lattice form. The shape of the protrusions may be variously modified within a range modifiable by those skilled in the art. By the protrusions formed on the outer surface of the body 310, the grip feel of the operator with respect to the marking apparatus 30 is improved, so that slipping of the marking apparatus 30 from the operator's hand can be minimized when performing scleral buckling, thereby further improving the efficiency of the surgery.

In addition, according to an embodiment, the marking apparatus 30 may be provided as a kit. Here, the marking apparatus 30 includes both the marking apparatus described with reference to FIGS. 3 to 7 and the marking apparatus described with reference to FIG. 8. More specifically, the marking apparatus 30 may be packaged in a sterilized state in a packaging member and provided as a kit. The marking apparatus 30 may be accommodated in a packaging member (e.g., a tray or the like). In addition, in the interior of the kit, a manual for the marking apparatus 30 may be further accommodated in addition to the marking apparatus 30.

The above detailed description is illustrative of the present invention. In addition, the above description represents and describes preferred embodiments of the present invention, and the present invention can be used in various other combinations, modifications, and environments. In other words, changes or modifications are possible within the scope of the inventive concept disclosed in the present specification, within the scope equivalent to the disclosure set forth, and/or within the scope of the skill or knowledge in the art. The embodiments set forth describe the best mode for implementing the technical idea of the present invention, and various changes required in the specific application fields and uses of the present invention are also possible. Therefore, the above detailed description of the invention is not intended to limit the present invention to the disclosed embodiments. In addition, the appended claims should be construed as including other embodiments as well.

### [Industrial Applicability]

According to the scleral marker and the marking apparatus including the same according to the embodiments of the present disclosure, a fixing member can be efficiently sutured at a break position of the retina.

## Claims

1. A marking apparatus comprising:
a body;
a connection part extending from the body in a longitudinal direction of the body; and
a marking part coupled to the connection part,
wherein the marking part includes a plurality of markers in contact with an eyeball, and the markers are disposed to be spaced apart from each other by a predetermined distance while forming an arc having a curvature corresponding to the eyeball.

2. The marking apparatus of claim 1, wherein the plurality of markers include a first marker and a second marker, and the first marker and the second marker are disposed in directions orthogonal to each other when viewed from above.

3. The marking apparatus of claim 2, wherein the first marker is disposed to be spaced apart in the longitudinal direction of the body, and the second marker is disposed to be spaced apart in a direction perpendicular to the longitudinal direction of the body when viewed from above.

4. The marking apparatus of claim 3, wherein the connection part includes:
a first connection part extending from one end of the body; and
a second connection part extending from the other end of the body, and
the marking part includes:
a first marker body connected to the first connection part and coupled to the first marker; and
a second marker body connected to the second connection part and coupled to the second marker.

5. The marking apparatus of claim 4, wherein the first marker body has a longitudinal direction parallel to the longitudinal direction of the body when viewed from above, and the second marker body has a longitudinal direction orthogonal to the longitudinal direction of the body when viewed from above.

6. The marking apparatus of claim 4, wherein the first marker is formed at a lower end of the first marker body, and the second marker is formed at an upper end of the second marker body.

7. The marking apparatus of claim 4, wherein the first marker includes a 1-1 marker, a 1-2 marker, and a 1-3 marker, the 1-1 marker is formed at a front end of the first marker body, the 1-3 marker is formed at a rear end of the first marker body, the 1-2 marker is formed between the 1-1 marker and the 1-3 marker, a straight-line distance between a tip of the 1-1 marker and a tip of the 1-2 marker is 5 mm, and a straight-line distance between the tip of the 1-2 marker and a tip of the 1-3 marker is 2.5 mm.

8. The marking apparatus of claim 7, wherein the second marker includes a 2-1 marker and a 2-2 marker, the 2-1 marker and the 2-2 marker are respectively formed at both ends of the second marker body, and a straight-line distance between a tip of the 2-1 marker and a tip of the 2-2 marker is 8 mm.

9. The marking apparatus of claim 1, wherein an end of the marker is formed to be flat.

10. The marking apparatus of claim 4, wherein each of the first connection part and the second connection part is formed in a shape whose diameter decreases with increasing distance from the body.

11. The marking apparatus of claim 1, wherein lattice-shaped protrusions are formed on an outer surface of the body.

12. The marking apparatus of any one of claims 1 to 11, wherein the apparatus is used in surgery including scleral buckling performed to reattach a detached retina.

13. The marking apparatus of any one of claims 1 to 11, wherein the marker is in contact with the sclera constituting the eyeball to mark positions at which needles are inserted in order to fix a fixing member at a break position.

14. A kit comprising:
the marking apparatus of any one of claims 1 to 11; and
a packaging member accommodating the marking apparatus.
